# EUROPÄISCHE PATENTANMELDUNG

(11) **EP 1 213 328 A1**
(43) Veröffentlichungstag der Anmeldung: **12.06.2002**
(21) Anmeldenummer: 00127005.7
(22) Anmeldetag: 08.12.2000
(51) Int. Cl.: C09B 19/00, G01N 33/533, C07D 265/34

(54) **Oxazinderivate**

(71) Anmelder: Evotec OAI AG, 22525 Hamburg (DE)
(72) Erfinder: Fries, Joachim, 25463 Tornesch (DE); Lopez-Calle, Eloisa, 20535 Hamburg (DE); Drexhage, Karl Heinz, Prof. Dr., 57076 Siegen (DE)
(74) Vertreter: Draudt, Jutta, Dr.

(57) **Zusammenfassung**

Es werden Oxazinderivate beschrieben, mit denen ein breites Spektrum von zu untersuchendem Material markiert und mittels Fluoreszenztechniken identifiziert werden kann.

## Beschreibung

Die vorliegende Erfindung betrifft Oxazinderivate, ein Verfahren zur ihrer Herstellung und ihre Anwendung als Fluoreszenzfarbstoffe zur Markierung von biologischem und nichtbiologischem Material im roten Spektralbereich.

Farbstoffe auf Oxazinbasis sind weit verbreitet. Man findet sie oftmals im Bereich der Textilindustrie zum Färben von Stoffen verschiedener Art. So beschreibt die EP-B-0 603 129 Oxazinfarbstoffe, die jeweils an den Benzolringen exoständige Aminogruppen, die substituiert sein können, aufweisen. Die schwer löslichen Salze dieser basischen Oxazinfarbstoffe eignen sich zum Färben oder zum Bedrucken von natürlichen Fasern und voll synthetischen Fasern.

Aus der US-A-5656759 ist ein kationischer Oxazinfarbstoff bekannt, der ebenfalls zwei exoständige Aminogruppen, die substituiert sein können, aufweist. Die Oxazinfarbstoffe, die durch Substitution anorganischer Anionen hydrophob gemacht werden können, werden insbesondere in der Farbschicht eines Farbbandes für den Thermotransferdruck verwendet.

Aus der EP-A-0 747 447 sind Oxazinfarbstoffe bekannt, die als Fluoreszenzmarker in Verbindung mit biologischen Molekülen verwendet werden. Der Absorptionsbereich dieser Konjugate liegt bei 645 - 700 nm und entspricht dem roten Spektralbereich. Die kupplungsfähigen Oxazinderivate sind mindestens tetrazyklische Systeme, in denen mindestens einer der beiden exozyklischen Stickstoffe in die Ringstruktur integriert ist. Auf Grund des komplexen polyzyklischen Grundgerüsts ist allerdings der Aufwand zur Synthese dieser Farbstoffe sehr hoch.

Insbesondere für das Hochdurchsatzscreening mit konfokaler Fluoreszenzspektroskopie werden Farbstoffe benötigt, die sich durch eine hohe Fluoreszenzquantenausbeute, hohe Wasserlöslichkeit, hohe Fotostabilität sowie die Fähigkeit, an biologische und nicht-biologische Materialien bzw. Moleküle zu binden, auszeichnen.

Durch den kostengünstigen Zugang zu rotemittierenden LaserLichtquellen sowie die geringe Hintergrundfluoreszenz von biologischem Material bei roter Anregung gegenüber energiereicheren Anregungswellenlängen kommt dem Einsatz von rot-anregbaren Fluoreszenzfarbstoffen, zu denen unter anderem auch die Oxazin-Farbstoffe zählen, steigende Bedeutung zu.

Die Aufgabe der vorliegenden Erfindung besteht darin, rotanregbare Fluoreszenzfarbstoffe zur Verfügung zu stellen, die gut wasserlöslich sind, wobei die photochemischen Eigenschaften erhalten bleiben. Des weiteren soll der Farbstoff in der Weise aktiviert sein, dass er zur Anknüpfung an biologische und nicht-biologische Materialien bzw. Moleküle befähigt ist, ohne dass es zu einer negativen Wechselwirkung mit den zu markierenden Materialien bzw. Molekülen kommt.

Diese Aufgabe wird mit den Oxazinderivaten gelöst, die in Patentanspruch 1 definiert sind.

Gegenstand der Erfindung sind Oxazinderivate der allgemeinen Formel I 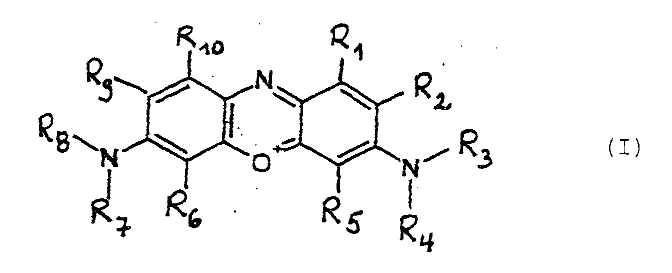 worin
(a) die Reste R₁, R₂, R₃, R₄, R₅, R₆, R₇, R₈, R₉ und R₁₀ voneinander unabhängig Wasserstoff, Alkyl, Cycloalkyl, Alkenyl, Alkenoxy, Aryl, Aryldiazo, Alkylaryl, Arylalkoxyl, Alkoxy, Alkoxycarbonyl, Hydroxy, Halogen, Cyan, Carbonyl, Acyl, Acyloxy, Carboxyl, Carbonamid, Halogencarbonamid, Sulfonyl, Sulfonylhalogenid, Säureester, Säureanhydrid, Säurehalogenid, Imid, Imidylester, Isothiocyanat, Azid, Dithionicotin-Derivat oder Amin bedeuten und gegebenenfalls substituiert sind,
(b) R₁ mit R₂ und/oder R₉ mit R₁₀ eine gesättigte oder ungesättigte C3- oder C4-Brücke bilden kann/können, die gegebenenfalls substituiert ist/sind;
(c) die Substituenten unabhängig voneinander Alkyl, Cycloalkyl, Alkenyl, Alkenoxy, Aryl, Aryldiazo, Alkylaryl, Arylalkoxyl, Alkoxy, Alkoxycarbonyl, Hydroxy, Halogen, Cyan, Carbonyl, Acyl, Acyloxy, Carboxyl, Carbonamid, Halogencarbonamid, Sulfonyl, Sulfonylhalogenid, Säureester, Säureanhydrid, Säurehalogenid, Imid, Imidylester, Isothiocyanat, Azid, Dithionicotin-Derivat oder Amin bedeuten können; und
(d) mindestens einer der Reste R₁, R₂, R₃, R₄, R₅, R₆, R₇, R₈, R₉ oder R₁₀ mindestens eine reaktive Gruppe für die Bindung an ein zu untersuchendes Material umfasst, oder mindestens eine reaktive Gruppe zusätzlich besitzt, oder ein Salz davon.

Die Unteransprüche betreffen bevorzugte Ausführungsformen der erfindungsgemäßen Oxazinderivate.

Im erfindungsgemäßen Sinne können die Reste R₁ bis R₁₀ die reaktive Gruppe darstellen und/oder diese enthalten, z. B. kann ein Substituent die reaktive Gruppe darstellen. Zusätzlich kann das Oxazinderivat eine reaktive Gruppe besitzen, wenn die Reste keine reaktive Gruppe umfassen. Diese reaktive Gruppe kann z. B. an die nicht-reaktiven Substituenten gebunden sein.

Die erfindungsgemäßen Oxazinderivate können als Tri- bzw. Tetra-/Pentazyklus vorliegen. In einer bevorzugten Ausführungsform bildet R₁ mit R₂ und/oder R₉ mit R₁₀ eine gesättigte oder ungesättigte C4-Brücke. Insbesondere bildet R₁ mit R₂ oder R₉ mit R₁₀ eine gesättigte oder ungesättigte C4-Brücke, womit ein weiterer Sechsring am Trizyklus entsteht, der gegebenenfalls substituiert ist. Ganz besonders bevorzugt bildet R₁ mit R₂ eine ungesättigte C₄-Brücke.

Die reaktive Gruppe dient dazu, den Farbstoff zu aktivieren, d. h. eine Stelle anzubieten, die eine Bindung zu dem zu untersuchenden Material schafft. Reaktive Gruppen im Sinne der Erfindung umfassen aktivierbare und aktivierte Gruppen. Die Bindung zum zu untersuchenden Material kann kovalenter oder nichtkovalenter Natur sein, wobei allerdings die kovalente Anknüpfung bevorzugt ist. Bevorzugt ist, dass nur eine reaktive Gruppe pro Farbstoffmolekül vorhanden ist. Die Aktivierung kann z. B. auch durch Licht erfolgen (Photoaktivierung).

Die reaktive Gruppe kann jede Gruppe sein, die zu einer Verknüpfung mit dem zu untersuchenden Material befähigt. Wenn das zu untersuchende Material beispielsweise ein Protein ist, dann bieten sich verschiedene Gruppen für die Anknüpfung an den Farbstoff an. Dazu zählen beispielsweise Amine aus Lysinresten oder Thiole aus freien Cystin- oder Cysteinresten.

Eine beispielhafte Aufzählung von bevorzugten reaktiven Gruppen umfasst Gruppierungen, wie ein Säureester, Säureanhydrid, Säurehalogenid, Imid, Imidylester, Carboxyl, Carbonamid, Halogencarbonamid, Sulfonylhalogenid, Isothiocyanat, Amin, Aryldiazo, Azid, Aryldiazo, Aldehyd, Keton oder Dithionicotin-Derivat.

Besonders bevorzugte reaktive Gruppen sind ein Säureester, Säureanhydrid, Säurehalogenid, Imid, Imidylester, Carboxyl, Carbonamid, Halogencarbonamid, Sulfonylhalogenid, Isothiocyanat, Amin, Azid, Aryldiazo oder Dithionicotin-Derivat.

Ganz besonders bevorzugte reaktive Gruppen sind ein N-Succinimidylester, der ggf. substituiert ist, Maleimid, Carboxyl, Halogenacetamid, Isothiocyanat, Aryldiazo, Azid, Sulfonylchlorid, Sulfo-Tetrafluorphenolester oder primäres bzw. sekundäres Amin.

Für den Fall, dass der N-Succinimidylester substituiert ist, ist ein bevorzugter Substituent die Sulfonsäuregruppe.

Es hat sich erfindungsgemäß herausgestellt, dass die Oxazinfarbstoffe ebenfalls eine Linkerverbindung enthalten können. Diese Linkerverbindung ist zwischen reaktiver Gruppe und einem nicht-brückebildenden Rest R₁, R₂, R₃, R₄, R₅, R₆, R₇, R₈, R₉ oder R₁₀ geschaltet. An die Linkerverbindung ist mindestens eine reaktive Gruppe gebunden. Die Linkerverbindungen werden eingeführt, wenn ein Bedarf dahingehend besteht, die reaktive Gruppe auf einfachem Weg zu verändern, d. h. die Funktionalität zu ändern. Beispielsweise kann mittels einer Linkerverbindung die Carbonsäure oder der N-Succinimidylester in ein Amin umgewandelt werden. Zum anderen ermöglichen es die Linkerverbindungen, den Abstand zwischen Farbstoff und dem zu untersuchenden Material zu variieren.

Zu geeigneten Linkerverbindungen zählen beispielsweise Polyoxyalkylverbindungen, aliphatische, cykloaliphatische oder aromatische Verbindungen. Sie können verzweigt oder unverzweigt sein und gegebenenfalls ungesättigte Einheiten und Heteroatome, z. B. Stickstoff, enthalten.

Die genannten Polyoxyalkyleinheiten sind polymere oder oligomere organische Reste, die über Sauerstoffbrücken miteinander verknüpft sind. Dazu zählen beispielsweise Polyether, Polyole, lösliche Carbohydrate, Derivate davon oder wasserlösliche Polymere.

Exemplarisch können Linkerverbindungen wie folgt dargestellt werden (Y bedeutet in den nachfolgenden Beispielen "reaktive Gruppe"):

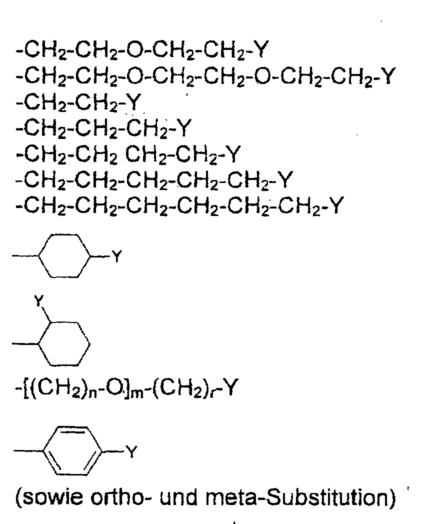

Die Reste und Substituenten Alkyl, Cycloalkyl, Alkenyl, Alkenoxy, Alkylaryl, Arylalkoxy, Alkoxy, Halogencarbonamid und Alkoxycarbonyl weisen in der Regel 1 bis 10, bevorzugt 1 bis 7 Kohlenstoffatome auf und können geradkettig oder verzweigt sein. Besonders bevorzugt sind 1 bis 4 Kohlenstoffatome. Bei Aryl handelt es sich vorzugsweise um Benzolringe.

Als Halogene kommen Fluor-, Chlor-, Brom- oder Jod in Frage.

Als Gegenion kann jedes zur Ladungsneutralisierung geeignete und mit dem kationischen Grundgerüst kompatible Anion verwendet werden, das die Wasserlöslichkeit der Gesamtverbindung nicht herabsetzt. Bevorzugt sind Halogenidionen, BF₄⁻ oder Tetraphenylborat. Besonders bevorzugt sind Chlorid- oder Bromidionen oder Tetraphenylborat.

Besonders bevorzugte Oxazinderivate sind spezielle Ausführungsformen, die durch die Formeln II bis V und VIII - IX wiedergegeben werden: 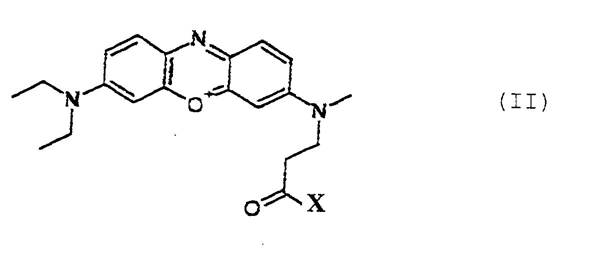 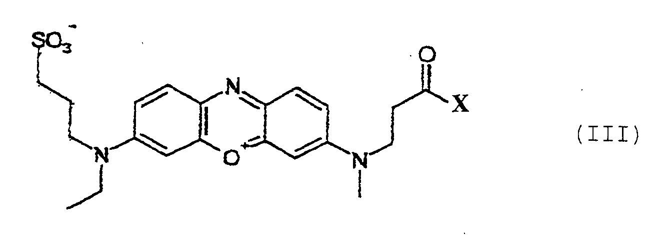 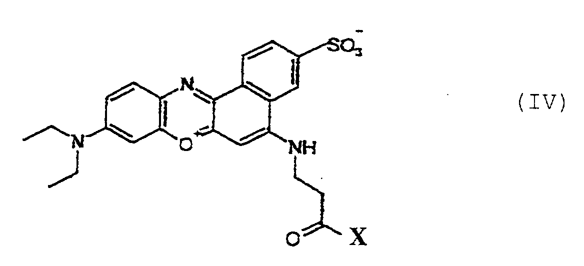 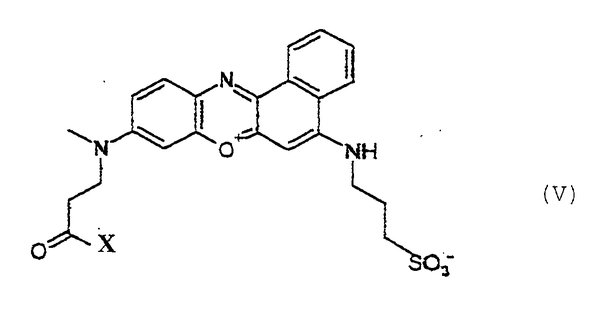 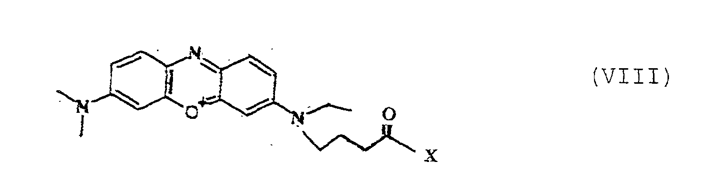 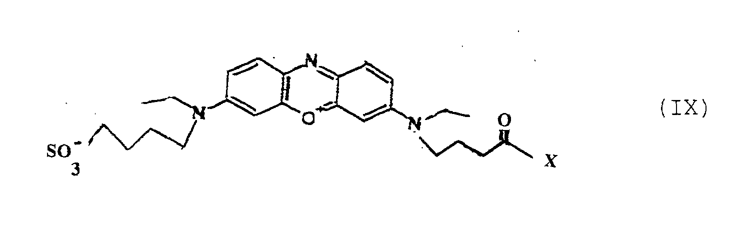 worin X OH oder eine N-Succinimidylestergruppe, die ggf. substituiert ist, bedeutet, wobei ebenfalls Salze davon eingeschlossen sind.

Die Vorteile der erfindungsgemäßen Oxazinfarbstoffe gegenüber den bisher bekannten UV-Farbstoffen, wie Cumarine oder Farbstoffen, die mit blauem oder grünen Licht angeregt werden, wie Fluoresceine und Rhodamine, sind die folgenden:
- hohe Reaktivität gegenüber Aminen, so dass ein großer Bereich von zu untersuchenden Substanzen auf einfache Weise kovalent markiert werden kann;
- hohe Wasserlöslichkeit, was zu einer minimalen Adsorption und einer minimalen unspezifischen Bindung führt;
- Anregung bei 630 bis 650 nm mittels kostengünstiger Laserdioden oder HeNe-Lasern;
- Emission mit einem Maximum im Bereich von 670 nm, wobei übliche Filteranordnungen verwendet werden können;
- hohe Fluoreszenzquantenausbeute, was klare und deutliche Fluoreszenzsignale hervorbringt;
- Lösungsmittelstabilität und pH-Stabilität (pH 1-10), so dass sie für sehr viele Anwendungen geeignet sind;
- neutrale Gesamtladung, was dazu führt, dass kein elektrostatischer Einfluß auf das markierte Molekül stattfindet;
- chemische Stabilität und daher eine außerordentliche Eignung in der organischen Chemie (Lösung und Festphase); und
- NIR-Anregbarkeit, was zu einer geringen Hintergrundfluoreszenz des zu untersuchenden Materials führt.

Am folgenden Beispiel der Proteinmarkierung soll ein weiterer entscheidender Vorteil gegenüber bekannten Markierungstechniken der erfindungsgemäßen Farbstoffe gezeigt werden. Durch herkömmliche Proteinmarkierungsverfahren, wie z.B. die Umsetzung einer aktivierten Carbonsäure mit Lys-Resten des Proteins bzw. von Maleimiden mit freien Thiol-Gruppen des Proteins werden immer mehrere dieser Gruppen des Proteins angesprochen, so dass es durch die Markierungsreaktion zu einer statistischen Verteilung der Markierung auf dem Protein kommt, d.h. einige Proteinmoleküle besitzen keinen, andere einen, zwei, drei oder mehrere Markierungen. In hochempfindlichen Fluoreszenztechniken, wie z. B. FCS (Fluorescence Correlation Spectroscopy) und FIDA (Fluorescence Intensity Distribution Analysis), kommt es durch diese heterogene Analytmischung auch zu heterogenen Signalverteilungen, die eine Auswertung der Daten erschwert. Darüber hinaus kommt es in Proteinen, die zwei, drei oder mehrere Label tragen, zu Farbstoff-Farbstoff-Wechselwirkungen, die das Fluoreszenzsignal drastisch abschwächen.

Mit den erfindungsgemäßen Oxazinderivaten ist es allerdings möglich, ein einfach markiertes zu untersuchendes Material für die Analyse zur Verfügung zu stellen. Damit ist gewährleistet, dass keine heterogene Signalverteilungen und Farbstoff-Farbstoff-Wechselwirkungen auftreten.

Die erfindungsgemäßen Oxazinderivate können zusätzlich mindestens einen Affinitätsmarker aufweisen, der eine einfache Reinigung durch Affinitätschromatographie ermöglicht. Der Affinitätsmarker kann z. B. an die Linkerverbindung gebunden sein.

In einer bevorzugten Ausführungsform weisen die Oxazinderivate nur einen Affinitätsmarker auf. Im Prinzip kann jeder Affinitätsmarker verwendet werden, der geeignet ist, in der nachfolgenden Affinitätschromatographie eine saubere Trennung durchzuführen. Als Beispiele können an dieser Stelle Affinitätsmarker, wie Biotin, Hexa-His oder Haptene genannt werden.

Ein Verfahren zur gezielten Markierung von zu untersuchendem Material mittels der affinitätsmarkierten Oxazinderivate kann wie folgt durchgeführt werden:
a) Bereitstellen einer Probe, umfassend das zu untersuchende Material;
b) Umsetzung des zu untersuchenden Materials mit einem oben beschriebenen, einen Affinitätsmarker enthaltenden Oxazinderivat, wobei sich ein markiertes zu untersuchendes Material bildet, das als Mischung kein, ein oder mehrere Oxazinderivate als Markierung aufweist; und
c) Auftrennung des zu untersuchenden Materials in nicht, einfach oder mehrfach markierte Bestandteile durch Affinitätschromatographie.

Nachfolgend wird beispielhaft eine derartige Affinitätsaufreinigung mit dem System Biotin/Avidin (Affinitätsmarker/Trägermaterial) vorgestellt.

Durch den Einsatz des unten dargestellten affinitätsmarkierten erfindungsgemäßen Farbstoff-Reaktiv-Moleküls kann der oben beschriebene Nachteil der bekannten Markierungstechniken umgangen werden. Dabei steht die Biotin-Einheit stellvertretend für einen beliebigen Affinitätsmarker, Oxazinderivat III für einen erfindungsgemäßen Farbstoff und der N-Succinimidylester für eine beliebige reaktive Gruppe. Ein solches Molekül reagiert analog dem herkömmlichen Markierungsverfahren mit dem Protein, was in einer heterogenenen Mischung wie oben beschrieben resultiert. Durch eine nachfolgende Affinitätsreinigung an einem geeigneten Trägermaterial (hier monomeres Avidin) werden unmarkierte Proteine aus der Probe entfernt. Durch nachfolgende vorsichtige Elution können einfachmarkierte Proteine eluiert werden, während doppel- und höhermarkierte Proteine aufgrund der kooperativ verstärkten Bindung an das Trägermaterial gebunden bleiben. Somit kann ein homogener Analyt mit exakt einem Farbstoffmarker pro Proteinmolekül erhalten werden.

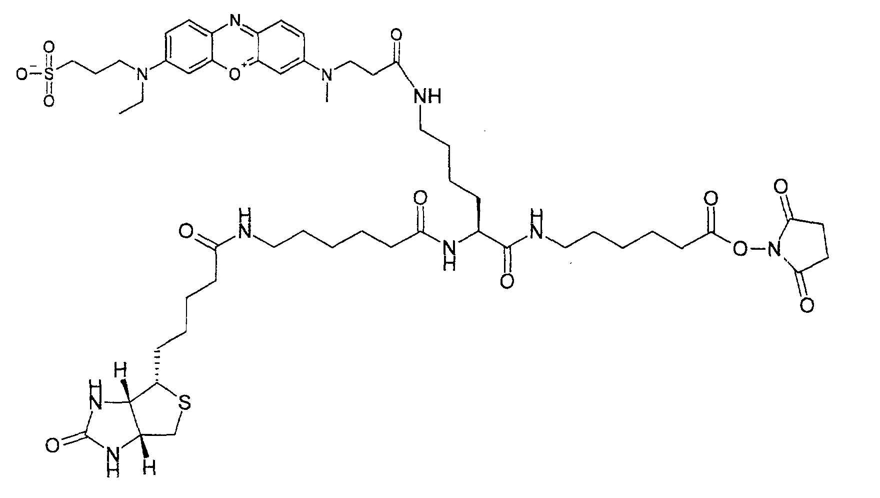

Das zu untersuchende Material kann kovalent oder nicht-kovalent an den Oxazinfarbstoff gebunden sein, wobei es allerdings bevorzugt kovalent gebunden ist. Bei dem zu untersuchenden Material handelt es sich vorzugsweise um ein biologisches Material, eine chemische Verbindung, insbesondere eine biologisch aktive Substanz und/oder einen pharmazeutischen Wirkstoff, einen synthetischen oder biologischen Mikropartikel oder eine Kombination davon.

Die chemischen Verbindungen können alle synthetischen Verbindungen umfassen, die als ein einzelnes Molekül oder Aggregat, beispielsweise als kleine organische Verbindung und auch als Oligomer oder Polymer vorliegen. Vorzugsweise handelt es sich bei den chemischen Verbindungen um biologisch aktive Substanzen und/oder pharmazeutische Wirkstoffe.

Synthetische Mikropartikel, d.h. insbesondere lösliche und suspendierbare Trägermaterialien, bezeichnen jede Art von synthetischen Mikropartikeln, die in einer Flüssigkeit, insbesondere einer wässrigen Lösung, gelöst oder suspendiert werden können. Die Durchmesser d der Mikropartikel sind gleich oder kleiner als 1 µm, bevorzugt liegen sie im Bereich von 1 nm ≤ d ≤ 1 µm, besonders bevorzugt im Bereich von 10 nm ≤ d ≤ 500 nm, ganz besonders bevorzugt im Bereich von 50 nm ≤ d ≤ 300 nm. Beispiele für die synthetischen Mikropartikel umfassen solche aus organischen Polymeren. Zum Beispiel können Polymere auf Dendrimer-Basis, bevorzugt Polyethylenglykol auf Dendrimer-Basis verwendet werden. Es können jedoch alle synthetischen Mikropartikel verwendet werden, die unter den experimentellen Bedingungen löslich oder suspendierbar sind, insbesondere Glaspartikel mit definierter Porengröße, Cellulose-, Silikagel- und andere Typen von Polystyrolpartikeln, letztere gegebenenfalls vernetzt mit Divinylbenzol und/oder gepropft mit Polyethylenglykol und/oder funktionalisiert mit Amino, Hydroxy, Carboxyl oder Halogen. Weitere Beispiele von möglichen synthetischen Mikropartikeln umfassen gepropfte Co-Polymer-, Polyacrylamid-, Latex-, gegebenenfalls mit N,N'-bis-Acryloylethylendiamin gepropfte Dimethylacrylamidpartikel und polymerüberzogene Glaspartikel. Bei den biologischen Mikropartikeln handelt es sich vorzugsweise um vesikuläre Partikel oder virus-ähnliche Partikel.

Bei dem biologischen Material kann es sich beispielsweise um Nukleotide, Oligonukleotide, Zucker, Lipide, Membranen, Zellen, Zellbestandteile, DNA, RNA, Peptide, Proteine, Antikörper, Haptene oder Antigene handeln.

Die vorliegende Erfindung betrifft auch ein Kit zur Markierung eines, wie oben beschriebenen, zu untersuchenden Materials. Dieses Kit umfasst, neben üblichen Bestandteilen, ein Oxazinderivat nach der allgemeinen Formel I. Übliche Bestandteile sind z. B. Puffer, Analysegefäße und Instruktionen zur Durchführung der Untersuchungen. In einer bevorzugten Ausführungsform enthält das Kit ein Oxazinderivat der Formel II, III, IV, V , VIII oder IX, insbesondere in Form eines N-Succinimidylesters, der ggf. substituiert sein kann.

Das zu untersuchende Material ist vorzugsweise ein biologisches Material, eine chemische Verbindung, insbesondere eine biologisch aktive Substanz und/oder ein pharmazeutischer Wirkstoff, ein synthetischer oder biologischer Mikropartikel oder eine Kombination davon. Die einzelnen Materialien sind bereits oben beschrieben worden.

Die erfindungsgemäßen Oxazinderivate eignen sich hervorragend für chemische oder biotechnische Untersuchungen. Dazu zählen: die Identifizierung und Charakterisierung von biologischem Material oder chemischen Verbindungen, Suche nach biologisch aktiven Substanzen und/oder pharmazeutischen Wirkstoffen, die Identifizierung von Analyten in diagnostischen und/oder therapeutischen Verfahren, die Genomanalyse oder die Reinigung und Konzentrierung von Substraten.

Die genannten Untersuchungen werden bevorzugt mittels Laserlichtanalytik und Fluoreszenzdetektion durchgeführt.
Die dabei angewandten Messverfahren schließen praktisch alle Verfahren ein, mit denen das Oxazinderivat gemessen werden kann. Zu diesen Verfahren zählen die Spektrometrie, Mehrphotonenanregung, insbesondere Zwei-Photonen-Anregung, Laser-Scanning-Mikroskopie, Nahfeldspektroskopie, Photonenverteilungsanalysen, insbesondere FIDA und 2-D-FIDA, Fluoreszenz-Lebensdauer-Analyse und Fluoreszenz-Polarisationsanalyse.
Ganz besonders bevorzugt werden die Messungen unter Verwendung eines konfokalen Mikroskops oder anderen konfokalen Optiken durchgeführt.

Die Auswertung der Daten erfolgt schließlich vorzugsweise durch Autokorrelationsanalyse und /oder Kreuzkorrelationsanalyse.

Bei der 2-D-FIDA und Kreuz-Korrelationsanalyse wird ein Messvolumen einer Probe mit zwei Wellenlängen simultan bestrahlt und die Information über eine zeitliche Koinzidenzanalyse von zwei Farbstoffspezies, wie die erfindungsgemäßen Farbstoffe in Kombination mit grün anregbaren Fluoreszenzfarbstoffen, erhalten.

Ähnlich ist der Einsatz in Fluoreszenz-Resonanz-Energie-Transfer-Experimenten (FRET) von großem Nutzen, wobei die erfindungsgemäßen Oxazinderivate mit einer Reihe von bekannten Xanthenen als Paar für den Energie-Transfer verwendet werden.

Die erfindungsgemäßen Oxazinderivate können ebenfalls sehr gut in FISH-(Fluorescence-in-situ-hybridization) oder PCR-Verfahren (Polymerase-chain reaction) verwendet werden.

Die erfindungsgemäßen Oxazinfarbstoffe sind synthetisch einfach zugänglich, was sie von den wesentlich schwerer herstellbaren Verbindungen des Standes der Technik unterscheidet. Die erfindungsgemäßen Farbstoffe weisen alle eine sehr kompakte molekulare Struktur auf, so dass die Wechselwirkung mit dem zu untersuchenden Material als äußerst gering einzuschätzen ist. Insbesondere werden die biologischen Eigenschaften von biologischem Material nicht verändert. Die in der Regel neutrale Gesamtladung minimiert die elektrostatische Wechselwirkung zwischen dem Farbstoff und der markierten Komponente. Die erfindungsgemäßen Fluoreszenzfarbstoffe weisen eine hohe Reaktivität gegenüber den zu untersuchenden Materialien, insbesondere Aminen, eine hohe Fluoreszenzquantenausbeute, eine hohe Lösungsmittelbeständigkeit und pH-Beständigkeit, eine hohe chemische Stabilität und eine NIR-Anregbarkeit auf. Die Farbstoffe der vorliegenden Erfindung sind durch eine geringe Anregungsenergie und somit einer geringen Photozerstörung gekennzeichnet. Die besonderen Anregungs- und Emissionswellenlängen der erfindungsgemäßen Farbstoffe ermöglichen die Verwendung von kommerziell erhältlichen Filtersystemen. Vorteilhaft ist weiterhin die geringe Hintergrundfluoreszenz von chemischen und biologischen Materialien bei roter Anregung gegenüber energiereicheren Anregungwellenlängen. Die erfindungsgemäßen Farbstoffe sind somit sehr gut im roten Spetralbereich einsetzbar. Die hohe chemische Stabilität ist insbesondere unter Bedingungen der Festphasensynthese von Bedeutung. Die erfingungsgemäßen Farbstoffe zeigen eine geringe Adsorptions- oder unspezifische Bindungstendenz und sind sehr gut wasserlöslich. Sie sind somit ideal für chemische und biotechnische Untersuchungen in wässrigen Lösungen verwendbar.

Die erfindungsgemäßen Oxazinderivate sind beispielsweise auf folgendem Wege darstellbar:

Ein 3-Aminophenol der Formel VI 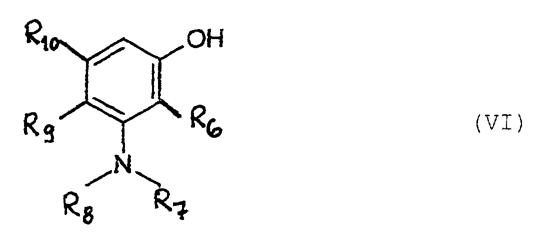 wird mit einer Nitrosophenylverbindung der Formel VII 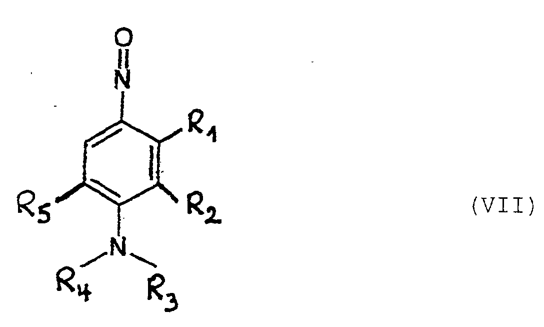 im sauren Medium bei mittlerer Hitze während eines Zeitraumes von 10 Minuten bis 5 Stunden umgesetzt. Die Reste R₁ bis R₁₀ haben die bereits oben angegebenen Bedeutungen.

Es hat sich herausgestellt, dass die Reaktion bei einer Temperatur im Bereich von 50 bis 70°C, vorzugsweise 55 bis 65°C, durchgeführt wird. Dazu wird die 3-Amino-Phenol-Verbindung in bspw. Eisessig gelöst, wonach innerhalb eines Zeitraumes im Bereich von 10 Minuten bis 5 Stunden die Nitrosoverbindung hinzugegeben wird. Nach Entstehen einer tiefblauen Lösung wird die Reaktion in der Regel beendet sein. Anschließend wird gereinigt, normalerweise über eine Säulenchromatographie.

Des Weiteren sind noch folgende Synthesemöglichkeiten eingeschlossen, in denen die Ausgangsverbindungen II die nachfolgend gezeigten Strukturen aufweisen:

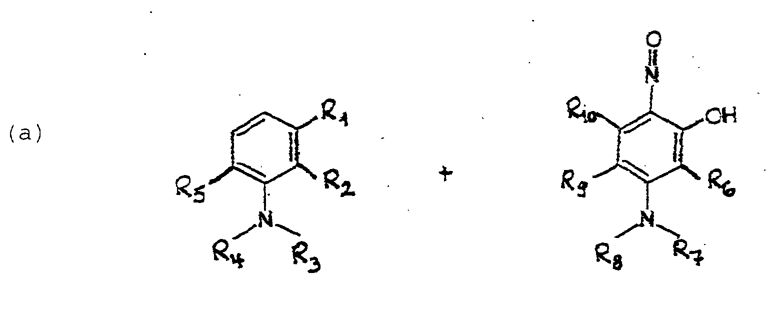

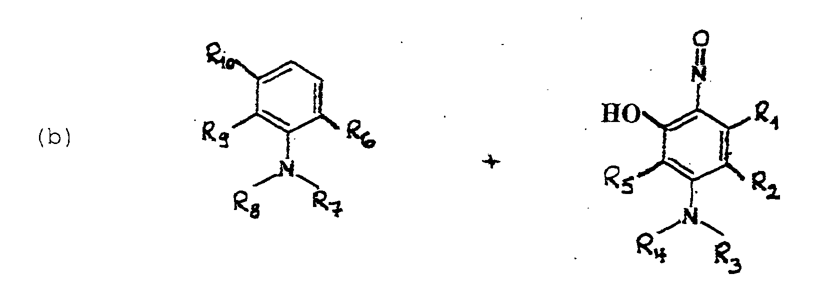

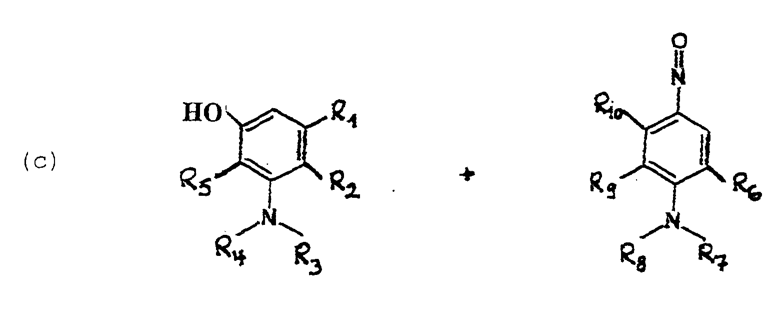

Die nachfolgenden Beispiele dienen zur Erläuterung der vorliegenden Erfindung.

### Beispiele

### Beispiel 1

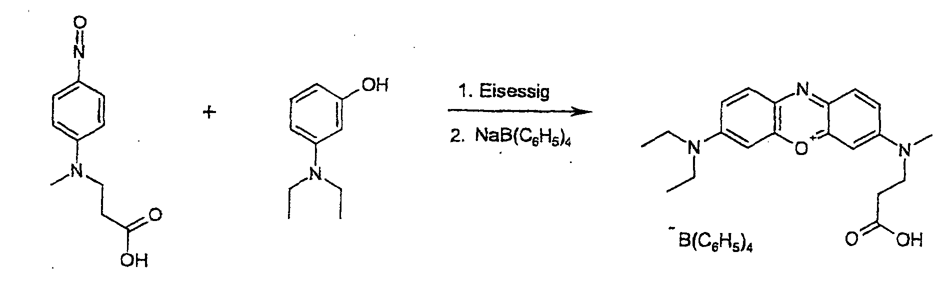

0,1 mol 3-Diethylaminophenol wurde in 20 ml Eisessig gelöst und auf 60°C erwärmt. Zu der Lösung wurde innerhalb von 30 Minuten 0,1 mol 3-(N-Methyl-N-(4-nitrosophenyl)-amino)-propionsäure in 10 ml Eisessig zugegeben. Die nun intensiv blaue Lösung wurde wurde 2 Stunden zum Rückfluss erhitzt. Nach Abdestillation des Eisessigs wurde der Rückstand säulenchromatographisch gereinigt (Eluent: Ethanol/H₂O: 5/1; Träger: SiO₂). Das bronzefarbene Produkt (2-Carboxy-ethyl)-methylamino)-diethylaminophenoxazin-5-ylium wurde in Wasser gelöst und mittels Natriumtetraphenylborat ausgefällt.

### Beispiel 2

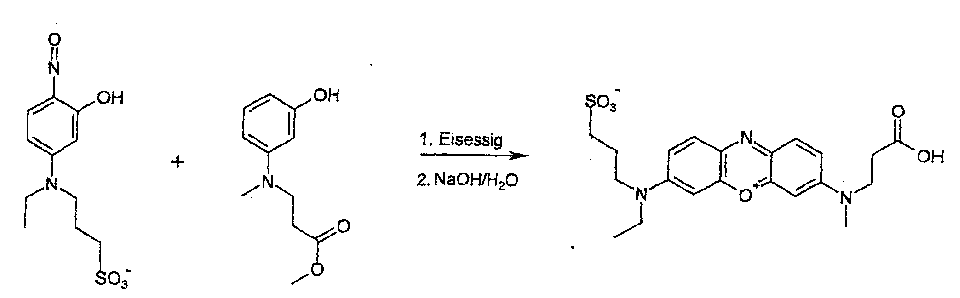

0,1 mol N-(3-Hydroxyphenyl)-N-methylamino-propionsäuremethylester wurde in 50 ml Eisessig gelöst und auf 60°C erwärmt. Zu dieser Lösung wurde innerhalb einer Stunde 0,1 mol 3-(N-ethyl-N-(4-nitroso-3-hydroxyphenyl)-amino)propan-sulfonsäure portionsweise zugegeben. Nach kurzer Zeit entstand eine tiefblaue Lösung. Nach dreistündiger Reaktionszeit wurde die Lösung eingeengt und säulenchromatgraphisch (Eluent: Ethanol; Träger: SiO₂) gereinigt. Das so erhaltene Produkt wurde in 1 N NaOH-Lösung aufgenommen und in der Siedehitze mehrere Stunden behandelt. Anschließend wurde die Lösung zur Trockne eingedampft und das Rohprodukt säulenchromatographisch (Eluent: Ethanol/H₂O: 10/1; Träger: SiO₂) separiert. Nach Umkristallisation aus Methanol wurden bronzefarbene Kristalle von [(2-Carboxy-ethyl)-methyl-amino]-[ethyl-(3-sulfo-propyl)-amino]-phenoxazin-5-ylium erhalten.

### Beispiel 3

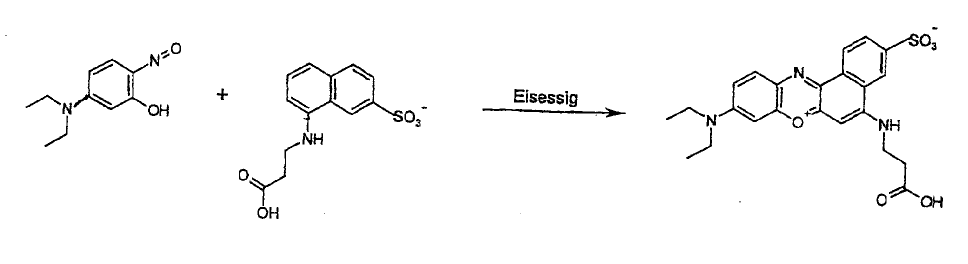

0,1 mol 3-(7-Sulfo-naphthylamino)-propionsäure und 0,1 mol 5-(Diethylamino)-2-nitroso-phenol wurden zusammen in 20 ml Eisessig 30 Minuten zum Rückfluss erhitzt. Beim Abkühlen der Reaktionslösung schieden sich grüne Kristalle von (2-Carboxyethylamino)-diethylamino-sulfobenzo[α]phenoxazin-7-ylium ab. Das Lösungsmittel wurde abgezogen und das Rohprodukt säulenchromatographisch (Eluent: Ethanol/Wasser: 20/1; Träger: SiO₂) aufgereinigt. Umkristallisation erfolgte aus Eisessig.

### Beispiel 4

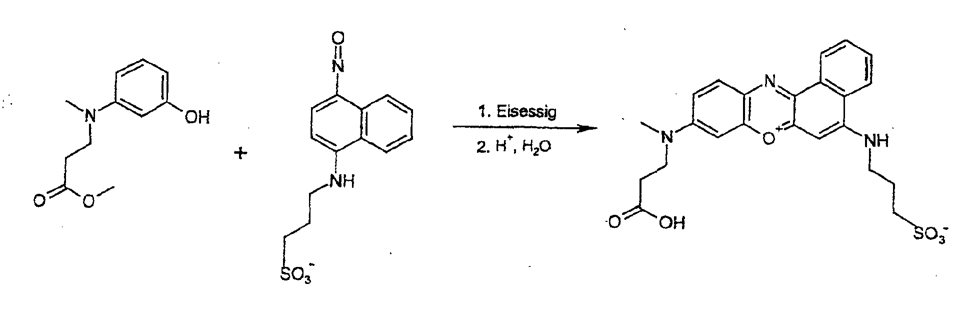

0,1 mol N-(4-Nitrosonaphtyl)-aminopropansulfonsäure und 0,1 mol N-(3-Hydroxyphenyl)-N-methylaminopropionsäuremethylester wurden zusammen in 20 ml Eisessig 30 Minuten zum Rückfluss erhitzt. Nach Abkühlen der Reaktionslösung wurde die Lösung zur Trockne eingedampft und der Rückstand aus Aceton umkristallisiert. Das erhaltene Produkt wurde in einem Gemisch aus 10 ml Wasser und 10 ml Aceton gelöst, mit 1 ml 1 N HCl-Lösung versetzt und 8 Stunden zum Rückfluss erhitzt. Anschließend wurde das Lösungsmittelgemisch abgezogen und das Rohprodukt säulenchromatographisch (Eluent: Ethanol/H₂O: 10/1; Träger SiO₂) separiert. Nach Umkristallisation aus Methanol wurden bronzefarbene Kristalle des (2-Carboxy-ethylamino)-ethyl-(3-sulfo-propyl)-aminobenzo[α]phenoxazin-7-ylium erhalten.
Ausbeute: 10 %

### Beispiel 5

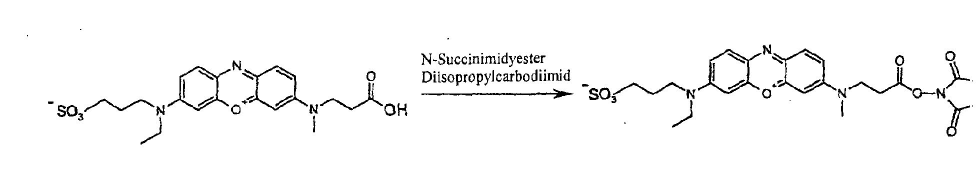

2 µmol [(2-Carboxy-ethyl)-methyl-amino]-[ethyl-(3-sulfo-propyl)-amino]-phenoxazin-5-ylium werden in 200 µl N,N-Dimethylformamid gelöst und eine Lösung von 10 µmol N-Hydroxysuccinimid in 100 µl N,N-Dimethylformamid sowie 2,4 µmol Diisopropylcarbodiimid zugegeben. Diese Lösung wird 3 Stunden bei Raumtemperatur geschüttelt und dann zur Trockne eingeengt. Der Umsatz zu [(2-Carboxy-ethyl)-methyl-amino]-[ethyl-(3-sulfo-propyl)-amino]-phenoxazin-5-ylium-succinimidylester ist > 90 %, daher ist eine weitere Aufreinigung nicht notwendig.

### Beispiel 6

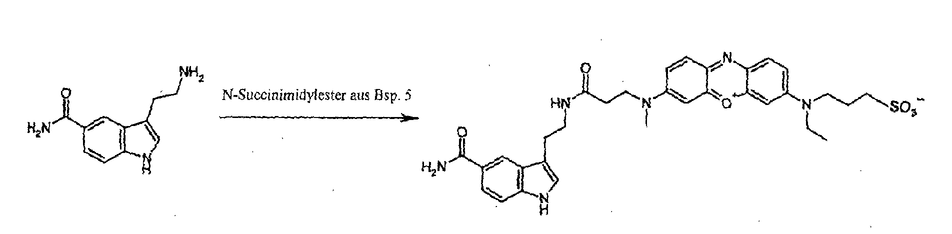

1 µmol 5-Carboxyamidotryptamin (5-CT) wird in 50 µl N,N-Dimethylformamid gelöst und einer Lösung von 1,5 umol [(2-Carboxy-ethyl)-methyl-amino]-[ethyl-(3-sulfo-propyl)-amino]-phenoxazin-5-ylium-succinimidylester in 200 µl N,N-Dimethylformamid zugegeben. Dieser Lösung werden 250 µl 0,15 M Borat-Puffer pH 8,6 zugesetzt und die Reaktionslösung 3 Stunden bei Raumtemperatur geschüttelt. Danach wird die Lösung zur Trockne eingeengt und das Rohprodukt säulenchromatographisch (Eluent: Methanol / Wasser Gradient: 0 % Methanol bis 100 % Methanol in 30 Minuten; Träger: Reversed Phase C18) aufgereinigt.

### Beispiel 7

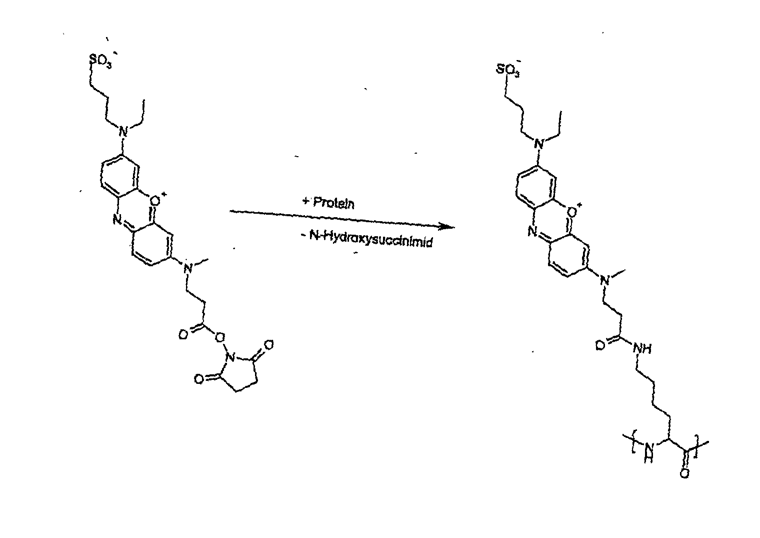

10 nmol Bovine IgG werden in 150 µl Wasser gelöst. Diese Lösung wird mit 15 µl 1 M Natriumcarbonatlösung pH 9,3 und 331,4 µl 0,1 M Natriumcarbonatlösung pH 9,3 sowie einer Lösung von 50 nmol [(2-Carboxy-ethyl)-methyl-amino]-[ethyl-(3-sulfo-propyl)-amino]-phenoxazin-5-ylium-succinimidylester in 3,6 µl N,N-Dimethylformamid versetzt und die Reaktionslösung 1 Stunde bei Raumtemperatur geschüttelt. Dann wird die Reaktion durch Zugabe von 50 µl 1,5 M Hydroxylaminlösung abgestoppt. Das Rohprodukt wird durch Ausschlusschromatographie (Eluent: PBS pH 7,4) aufgereinigt.

### Beispiel 8

Einführung eines Linkers in Oxazinderivat (III) 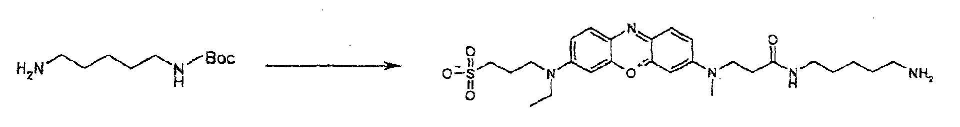
1. 2,0 eq Boc-cadaverin
   1,0 eq Oxazinderivat (III)
   2,0 eq PyBOP
   5,0 eq DIPEA
2. 20% TFA in DCM
2 µmol [(2-Carboxy-ethyl)-methyl-amino]-[ethyl-(3-sulfo-propyl)-amino]-phenoxazin-5-ylium (Oxazinderivat III) werden in 200 µl N,N-Dimethylformamid gelöst und eine Lösung von 4µmol mono-t-Butoxycarbonyl-1,5-diaminopentan (Boc-cadaverin) in 50 µl N,N-Dimethylformamid, eine Lösung von 4 umol Benzotriazol-1-yl-oxytris-pyrrolidino-phosphonium Hexafluorophosphat (PyBOP) in 50 µl N,N-Dimethylformamid sowie 10 µmol N-Ethyl-diisopropyl-amin (DIPEA) zugegeben. Diese Lösung wird 16 Stunden bei Raumtemperatur geschüttelt und dann zur Trockne eingeengt. Der Rückstand wird mit 300 µl einer Lösung von 20 % Trifluoressigsäure (TFA) in Dichlormethan (DCM) versetzt und 30 Minuten bei Raumtemperatur geschüttelt. Die Lösung wird zur Trockne eingeengt und das Rohprodukt säulenchromatographisch über HPLC aufgereinigt.

### Beispiel 9

Einführung eines Linkers in Oxazinderivat (III) 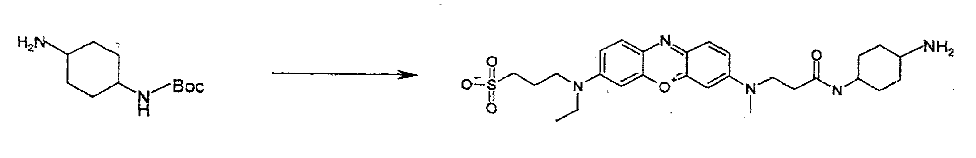
1. 2,0 eq Boc-CHA
   1,0 eq Oxazinderivat (III)
   1,0 eq PyBOP
   4,0 eq DIPEA
2. 20% TFA in DCM
2 µmol [(2-Carboxy-ethyl)-methyl-amino]-[ethyl- (3-sulfo-propyl) - amino]-phenoxazin-5-ylium(Oxazinderivat III) werden in 200 µl N,N-Dimethylformamid gelöst und eine Lösung von 4 µmol mono-t-Butoxycarbonyl-trans-1,4-diaminocyclohexan (Boc-CHA) in 50 µl N,N-Dimethylformamid, eine Lösung von 2 µmol Benzotriazol-1-yloxy-tris-pyrrolidino-phosphonium Hexafluorophosphat (PyBOP) in 50 µl N,N-Dimethylformamid sowie 8 µmol N-Ethyl-diisopropyl-amin (DIPEA) zugegeben. Diese Lösung wird 16 Stunden bei Raumtemperatur geschüttelt und dann zur Trockne eingeengt. Der Rückstand wird mit 300 µl einer Lösung von 20 % Trifluoressigsäure (TFA) in Dichlormethan (DCM) versetzt und 30 Minuten bei Raumtemperatur geschüttelt. Die Lösung wird zur Trockne eingeengt und das Rohprodukt säulenchromatographisch über HPLC aufgereinigt.

### Beispiel 10

### Affinitätsmarkierung

1 mmol N-ε-(9-Fluorenylmethoxycarbonyl)-aminohexansäure wurden in 1200 µl Dichlormethan und 300 µl N,N-Dimethylformamid (DMF) gelöst und auf 0°C abgekühlt. 500 µmol N,N'-Diisopropylcarbodiimid wurden in 80 µl N,N-Dimethylformamid gelöst und zur Lösung der Aminosäure hinzugegeben. Die Reaktionslösung wurde 30 Minuten bei 0°C stehengelassen und anschließend das Dichlormethan im Vakuum entfernt. Der Rückstand wurde mit 1000 µl N,N-Dimethylformamid aufgenommen, wobei eine klare Lösung erhalten wurde. Diese Lösung wurde zu 100 µmol Wang Harz (Wang Resin) in einer 5 ml Spritze mit Fritte gegeben. Zu der Suspension wurden 10 µmol Dimethylaminopyridin in 25 µl N,N-Dimethylformamid gegeben, die Mischung wurde über Nacht stehengelassen.

Das Harz wurde anschließend sechsmal mit 2 ml N,N-Dimethylformamid und dreimal mit 2 ml Dichlormethan und sechsmal mit 2 ml *t*-Butylmethylether gewaschen, getrocknet und zur Bestimmung der Beladung ausgewogen. Die bestimmte Beladung des Harzes betrug 0.8 mmol/g Harz. Zur weiteren Beladung wurde zunächst die 9-Fluorenylmethoxycarbonyl-Schutzgruppe (FMOC) mittels zweimaliger Behandlung für 5 bzw. 15 Minuten mit einer Lösung von 20 % Piperidin in N,N-Dimethylformamid abgespalten. Das Harz wurde danach sechsmal mit 2 ml N,N-Dimethylformamid gewaschen. 1 mmol N-α-(9-Fluorenylmethoxycarbonyl)-ε-(4-methyltrityl)-Llysin, gelöst in 120 µl N,N-Dimethylformamid sowie 1 mmol 1-[Bis(dimethylamino)methyliumyl]-1H-1,2,3-triazolo[4,5-*b*]pyridin-3-oxid hexafluorophosphat gelöst in 120 µl N,N-Dimethylformamid wurden zusammen mit 2 mmol N,N-Diisopropylethylamin zum Harz gegeben. Die Reaktionslösung wurde nach 30 Minuten abgezogen und der Vorgang der Beladung unter identischen Bedingungen nochmals wiederholt. Nach sechsmaligem Waschen des Harzes mit je 2 ml N,N-Dimethylformamid wurde die 9-Fluorenylmethoxycarbonyl Schutzgruppe mittels zweimaliger Behandlung für 5 bzw. 15 Minuten mit einer Lösung von 20 % Piperidin in N,N-Dimethylformamid abgespalten. Das Harz wurde danach wiederum sechsmal mit 2 ml N,N-Dimethylformamid gewaschen. Anschließend erfolgte die Umsetzung mit 250 µmol Biotinoylaminohexanoyl-N-hydroxy-succinimidylester in 300 µl N,N-Dimethylformamid. Nach 10 stündiger Reaktion wurde die Reaktionslösung abgesaugt und das Harz sechsmal mit 2 ml N,N-Dimethylformamid und dreimal mit 2 ml Dichlormethan und sechsmal mit 2 ml *t*-Butylmethylether gewaschen und getrocknet.

Zur Beladung mit dem Fluoreszenzfarbstoff (dye) wurden 10 µmol des Harzes abgenommen und in eine 5 ml Spritze überführt. Nachdem das Harz in 2 ml Dichlormethan vorgequollen wurde, erfolgte die Abspaltung der 4-Methyltrityl-Schutzgruppe (MTT) mittels fünfmaliger jeweils dreiminütiger Behandlung mit je 2 ml 30 % Hexafluorisopropanol (HFIP) in Dichlormethan. Danach wurde das Harz sechsmal mit 2 ml Dichlormethan und dreimal mit 2 ml N,N-Dimethylformamid gewaschen. Anschließend erfolgte die Umsetzung mit 25 µmol [(2-Carboxy-ethyl)-methyl-amino]-ethyl-(3-sulfo-propyl)-amino]-phenoxin-5-ylium-succinimidylester in 300 µl N,N-Dimethylformamid für drei Stunden bei Raumtemperatur. Nach erfolgter Reaktion wurde das Harz sechsmal mit 2 ml N,N-Dimethylformamid, sechsmal mit 2 ml Dichlormethan, sechsmal mit 2 ml Methanol und sechsmal mit 2 ml *t*-Butylmethylether gewaschen und getrocknet. Das Produkt wurde nunmehr mittels einer Mischung von 95 % Trifluoressigsäure (TFA), 2,5 % Wasser und 2,5 % Triispropylsilan (TIS) vom Harz abgespalten. Nach zweistündiger Reaktionszeit wurde die Lösung vom Harz stündiger Reaktionszeit wurde die Lösung vom Harz abfiltriert und im Vakuum eingeengt. Das als Rückstand verbleibende Rohprodukt wurde anschließend in Methanol aufgenommen und chromatographisch aufgereinigt.

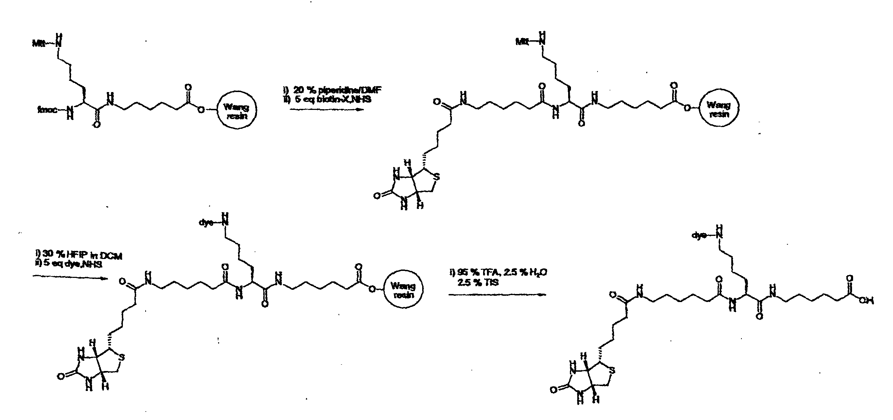

## Patentansprüche

1. Oxazinderivat der allgemeinen Formel (I) 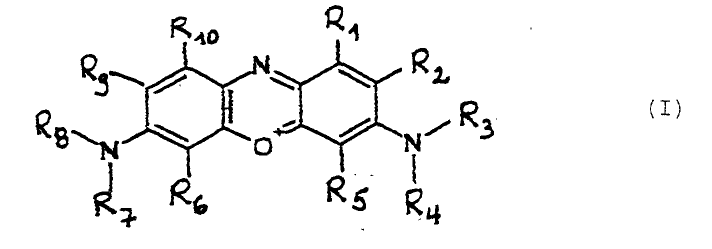 worin:
(a) die Reste R₁, R₂, R₃, R₄, R₅, R₆, R₇, R₈, R₉ und R₁₀ voneinander unabhängig Wasserstoff, Alkyl, Cycloalkyl, Alkenyl, Alkenoxy, Aryl, Aryldiazo, Alkylaryl, Arylalkoxyl, Alkoxy, Alkoxycarbonyl, Hydroxy, Halogen, Cyan, Carbonyl, Acyl, Acyloxy, Carboxyl, Carbonamid, Halogencarbonamid, Sulfonyl, Sulfonylhalogenid, Säureester, Säureanhydrid, Säurehalogenid, Imid, Imidylester, Isothiocyanat, Azid, Dithionicotin-Derivat oder Amin bedeuten und gegebenenfalls substituiert sind,
(b) R₁ mit R₂ und/oder R₉ mit R₁₀ eine gesättigte oder ungesättigte C3- oder C4-Brücke bilden kann/können, die gegebenenfalls substituiert ist/sind;
(c) die Substituenten unabhängig voneinander Alkyl, Cycloalkyl, Alkenyl, Alkenoxy, Aryl, Aryldiazo, Alkylaryl, Arylalkoxyl, Alkoxy, Alkoxycarbonyl, Hydroxy, Halogen, Cyan, Carbonyl, Acyl, Acyloxy, Carboxyl, Carbonamid, Halogencarbonamid, Sulfonyl, Sulfonylhalogenid, Säureester, Säureanhydrid, Säurehalogenid, Imid, Imidylester, Isothiocyanat, Azid, Dithionicotin-Derivat oder Amin bedeuten können; und
(d) mindestens einer der Reste R₁, R₂, R₃, R₄, R₅, R₆, R₇, R₈, R₉ oder R₁₀ mindestens eine reaktive Gruppe für die Bindung an ein zu untersuchendes Material umfasst, oder mindestens eine reaktive Gruppe zusätzlich besitzt, oder ein Salz davon.

2. Oxazinderivat nach Anspruch 1, **dadurch gekennzeichnet, dass** R₁ mit R₂ und/oder R₉ mit R₁₀ eine gesättigte oder ungesättigte C4-Brücke bildet.

3. Oxazinderivat nach Anspruch 2, **dadurch gekennzeichnet, dass** R₁ mit R₂ oder R₉ mit R₁₀ eine gesättigte oder ungesättigte C4-Brücke bildet.

4. Oxazinderivat nach mindestens einem der Ansprüche 1 bis 3, **dadurch gekennzeichnet, dass** R₃, R₄, R₇ oder R₈ eine reaktive Gruppe umfasst oder besitzt.

5. Oxazinderivat nach mindestens einem der Ansprüche 1 bis 4, **dadurch gekennzeichnet, dass** die reaktive Gruppe ein Säureester, Säureanhydrid, Säurehalogenid, Imid, Imidylester, Carboxyl, Carbonamid, Halogencarbonamid, Sulfonylhalogenid, Isothiocyanat, Amin, Azid, Aryldiazo, Aldehyd, Keton oder Dithionicotin-Derivat ist.

6. Oxazinderivat nach Anspruch 5, **dadurch gekennzeichnet, dass** die reaktive Gruppe ein Säureester, Säureanhydrid, Säurehalogenid, Imid, Imidylester, Carboxyl, Carbonamid, Halogencarbonamid, Sulfonylhalogenid, Isothiocyanat, Amin, Azid, Aryldiazo oder Dithionicotin-Derivat ist.

7. Oxazinderivat nach Anspruch 6, **dadurch gekennzeichnet, dass** die reaktive Gruppe ein N-Succinimidylester, der ggf. substituiert ist, Maleimid, Carboxyl, Halogenacetamid, Isothiocyanat, Aryldiazo, Azid, Sulfonylchlorid, Sulfo-Tetrafluorphenolester oder primäres bzw. sekundäres Amin ist.

8. Oxazinderivat nach Anspruch 7, **dadurch gekennzeichnet, dass** der N-Succinimidylester mit einer Sulfonsäuregruppe substituiert ist.

9. Oxazinderivat nach mindestens einem der Ansprüche 1 bis 8, **dadurch gekennzeichnet, dass** zwischen reaktiver Gruppe und einem nicht-brückebildenden Rest R₁, R₂, R₃, R₄, R₅, R₆, R₇, R₈, R₉ oder R₁₀ eine Linkerverbindung geschaltet ist.

10. Oxazinderivat nach Anspruch 9, **dadurch gekennzeichnet, dass** es sich bei den Linkerverbindungen um Polyoxyalkylverbindungen, aliphatische, cykloaliphatische oder aromatische Verbindungen handelt, die gegebenenfalls substituiert sein können.

11. Oxazinderivat nach Anspruch 1, **dadurch gekennzeichnet, dass** das Salz davon ein Gegenion enthält, das aus einem Halogenidion, einem BF₄⁻-Ion oder Tetraphenylborat gewählt ist.

12. Oxazinderivat nach Anspruch 11, **dadurch gekennzeichnet, dass** das Gegenion ein Chlorid- oder Bromidion oder Tetraphenylborat ist.

13. Oxazinderivat nach Anspruch 1, mit der Formel (II) 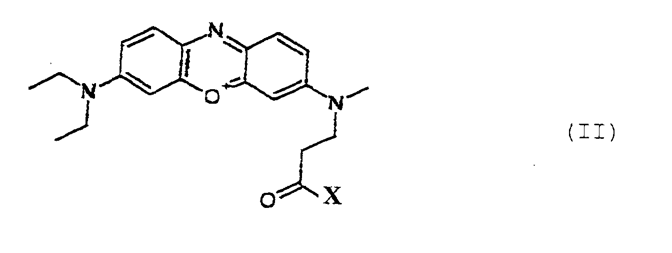 worin X OH oder eine N-Succinimidylestergruppe, die ggf. substituiert ist, bedeutet oder ein Salz davon.

14. Oxazinderivat nach Anspruch 1, mit der Formel(III) 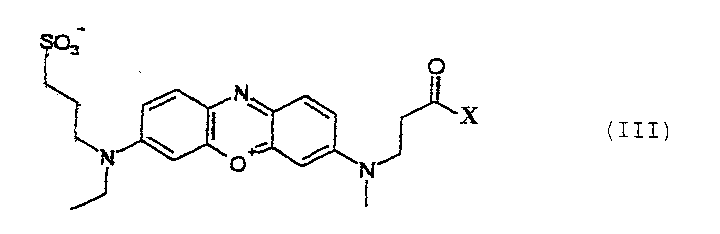 worin X OH oder eine N-Succinimidylestergruppe, die ggf. substituiert ist, bedeutet oder ein Salz davon.

15. Oxazinderivat nach Anspruch 1, mit der Formel (IV) 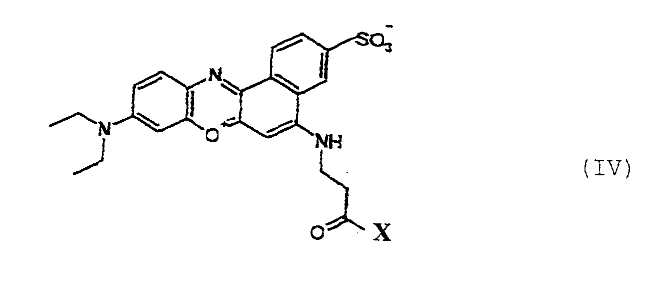 worin X OH oder eine N-Succinimidylestergruppe, die ggf. substituiert ist, bedeutet oder ein Salz davon.

16. Oxazinderivat nach Anspruch 1, mit der Formel (V) 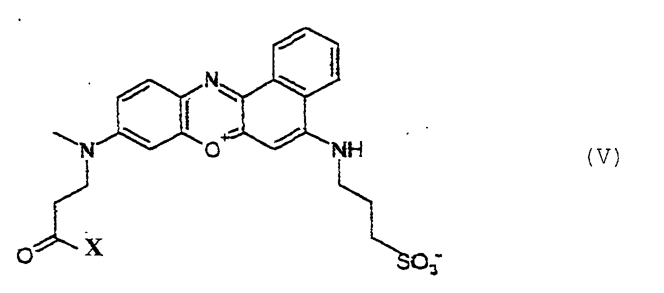 worin X OH oder eine N-Succinimidylestergruppe, die ggf. substituiert ist, bedeutet oder ein Salz davon.

17. Oxazinderivat nach einem der Ansprüche 13 bis 16, **dadurch gekennzeichnet, dass** das Salz davon ein Gegenion enthält, das aus einem Halogenidion, einem BF₄⁻-Ion oder Tetraphenylborat gewählt ist.

18. Oxazinderivat nach Anspruch 17, **dadurch gekennzeichnet, dass** das Gegenion ein Chlorid- oder Bromidion oder Tetraphenylborat ist.

19. Oxazinderivat nach mindestens einem der Ansprüche 1 bis 18, **dadurch gekennzeichnet, dass** die Derivate mindestens einen Affinitätsmarker aufweisen.

20. Oxazinderivat nach Anspruch 19, **dadurch gekennzeichnet, dass** die Derivate nur einen Affinitätsmarker aufweisen.

21. Oxazinderivat nach Anspruch 19 oder 20, **dadurch gekennzeichnet, dass** es sich bei den Affinitätsmarkern um Biotin, Hexa-His oder Haptene handelt.

22. Oxazinderivat nach mindestens einem der Ansprüche 1 bis 21, **dadurch gekennzeichnet, dass** die reaktive Gruppe an ein zu untersuchendes Material gebunden ist.

23. Oxazinderivat nach mindestens einem der Ansprüche 1 bis 22, **dadurch gekennzeichnet, dass** es sich bei dem zu untersuchenden Material um i) ein biologisches Material, ii) eine chemische Verbindung, insbesondere eine biologisch aktive Substanz und/oder einen pharmazeutischen Wirkstoff, iii) einen synthetischen oder biologischen Mikropartikel oder iv) eine Kombination davon handelt.

24. Oxazinderivat nach Anspruch 23, **dadurch gekennzeichnet, dass** es sich bei den synthetischen Mikropartikeln um lösliche oder suspendierbare Trägermaterialien handelt.

25. Oxazinderivat nach Anspruch 23, **dadurch gekennzeichnet, dass** es sich bei den biologischen Mikropartikeln um vesikuläre oder virus-ähnliche Partikel handelt.

26. Oxazinderivat nach Anspruch 23, **dadurch gekennzeichnet, dass** das biologische Material Nukleotide, Oligonukleotide, Zucker, Lipide, Membranen, Zellen, Zellbestandteile, DNA, RNA, Peptide, Proteine, Antikörper, Haptene oder Antigene umfasst.

27. Kit zur Markierung eines zu untersuchenden Materials, umfassend ein Oxazinderivat nach mindestens einem der Ansprüche 1 bis 26.

28. Kit nach Anspruch 27, **dadurch gekennzeichnet, dass** es ein Oxazinderivat nach mindestens einem der Ansprüche 13 bis 18 umfasst.

29. Verwendung eines Kits nach Anspruch 27 oder 28 oder eines Oxazinderivates nach mindestens einem der Ansprüche 1 bis 26 in chemischen oder biotechnischen Anwendungen.

30. Verwendung nach Anspruch 29, **dadurch gekennzeichnet, dass** das Kit oder das Oxazinderivat i) zur Identifizierung und Charakterisierung von biologischem Material oder chemischen Verbindungen, ii) zur Suche nach biologisch aktiven Substanzen und/oder pharmazeutischen Wirkstoffen, iii) zur Identifizierung von Analyten in diagnostischen und/oder therapeutischen Verfahren, iv) zur Genomanalyse oder v) zur Reinigung und Konzentrierung von Substraten verwendet wird.

31. Verfahren zur Herstellung der Oxazinderivate nach mindestens einem der Ansprüche 1 bis 26, bei dem man eine Verbindung der Formel VI 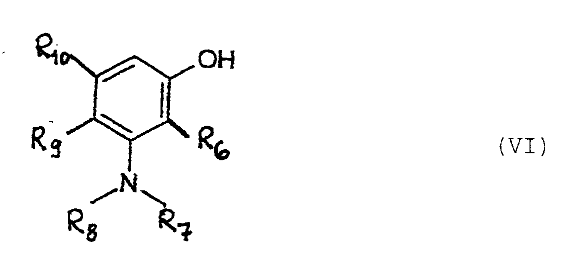 im sauren Medium bei mittlerer Hitze während eines Zeitraumes von 10 Minuten bis 5 Stunden mit einer Verbindung der Formel VII 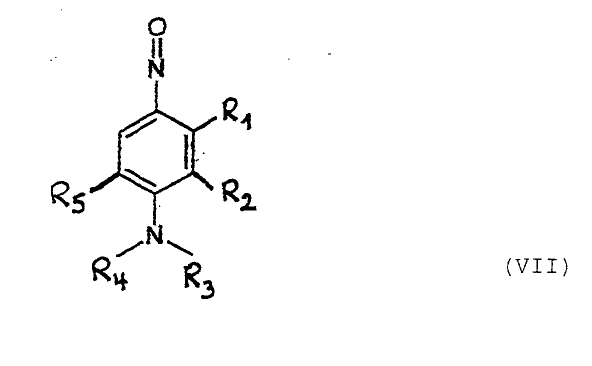 worin R₁ bis R₁₀ die in Anspruch 1 angegebenen Bedeutungen haben, umsetzt.

32. Verfahren nach Anspruch 31, **dadurch gekennzeichnet, dass** man als Ausgangsverbindungen folgende Aminoverbindungen und Nitrosoverbindungen verwendet:

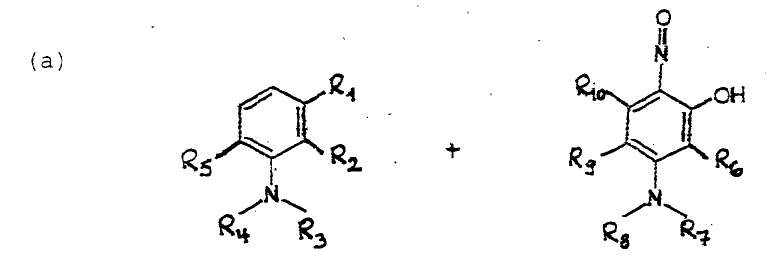

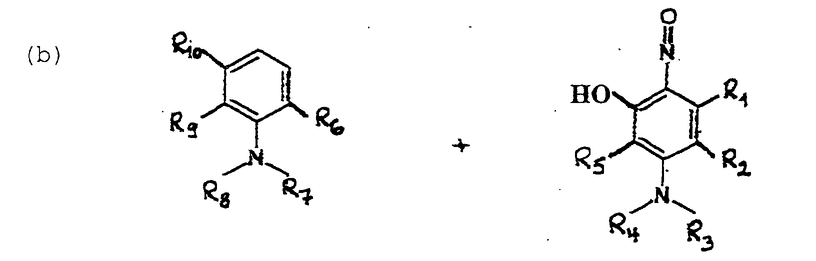

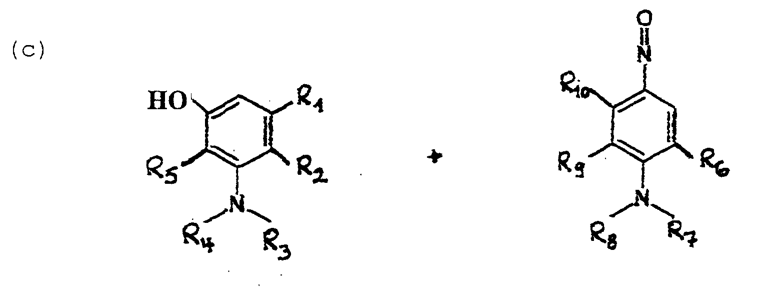

33. Verfahren nach Anspruch 31 oder 32, **dadurch gekennzeichnet, dass** das Oxazinderivat durch Umsetzung mit N-Succinimidylester in Gegenwart von N,N-Dimethylformamid und Diisopropylcarbodiimid aktiviert wird.

34. Verfahren zur gezielten Markierung von zu untersuchendem Material, **dadurch gekennzeichnet, dass** es die folgenden Schritte umfasst:
a) Bereitstellung einer Probe umfassend ein zu untersuchendes Material;
b) Umsetzung des zu untersuchenden Materials mit einem einen Affinitätsmarker enthaltenen Oxazinderivat gemäß mindestens einem der Ansprüche 17 bis 19, unter Bildung von markiertem zu untersuchendem Material, das als Mischung kein, ein oder mehrere Oxazinderivate als Markierung aufweist;
c) Auftrennung des zu untersuchenden Materials in nicht, einfach oder mehrfach markierte Oxazinderivate davon durch Affinitätschromatographie.

35. Verfahren nach Anspruch 34, **dadurch gekennzeichnet, dass** als Affinitätsmarker Biotin, Hexa-His oder Haptene verwendet werden.

36. Verfahren nach Anspruch 34 oder 35, **dadurch gekennzeichnet, dass** für die anschließende Untersuchung des Materials die Fraktion verwendet wird, die einfach markiert ist.
